# EUROPÄISCHE PATENTSCHRIFT

(11) **EP 2 024 059 B1**
(45) Veröffentlichungstag und Bekanntmachung des Hinweises auf die Patenterteilung: **09.05.2018**
(21) Anmeldenummer: 07729164.9
(22) Anmeldetag: 15.05.2007
(51) Int. Cl.: B01D 53/14, C07C 7/11

(54) **ENTFERNEN SAURER GASE AUS EINEM FLUIDSTROM BEI VERMINDERTER COABSORPTION VON KOHLENWASSERSTOFFEN UND SAUERSTOFF**
REMOVAL OF ACID GASES FROM A FLUID FLOW BY MEANS OF REDUCED COABSORPTION OF HYDROCARBONS AND OXYGEN
ÉLIMINATION DES GAZ ACIDES D'UN COURANT FLUIDIQUE AVEC COABSORPTION RÉDUITE DES HYDROCARBURES ET DE L'OXYGÈNE

(30) Priorität: 18.05.2006 EP 06114184
(43) Veröffentlichungstag der Anmeldung: 18.02.2009
(73) Patentinhaber: BASF SE, 67056 Ludwigshafen am Rhein (DE)
(72) Erfinder: WAGNER, Rupert, 67551 Worms (DE); LICHTFERS, Ute, 76187 Karlsruhe (DE); ASPRION, Norbert, 67063 Ludwigshafen (DE)
(74) Vertreter: BASF IP Association
(86) Internationale Anmeldenummer: PCT/EP2007/054716
(87) Internationale Veröffentlichungsnummer: WO 2007/135028

(56) Entgegenhaltungen:
- EP-A- 0 671 200
- EP-A1- 0 125 358
- US-A- 4 278 646

## Beschreibung

Die vorliegende Erfindung betrifft ein Verfahren zum Entfernen saurer Gase aus einem Fluidstrom und ein Absorptionsmittel.

Die Entfernung von Sauergasen, wie z.B. CO₂, H₂S, SO₂, CS₂, HCN, COS oder Merkaptanen, aus Fluidströmen, wie Erdgas, Raffineriegas, Synthesegas, ist aus unterschiedlichen Gründen von Bedeutung. Der Gehalt an Schwefelverbindungen von Erdgas muss durch geeignete Aufbereitungsmaßnahmen unmittelbar an der Erdgasquelle reduziert werden, denn die Schwefelverbindungen bilden in dem vom Erdgas häufig mitgeführten Wasser Säuren, die korrosiv wirken. Für den Transport des Erdgases in einer Pipeline müssen daher vorgegebene Grenzwerte der schwefelhaltigen Verunreinigungen eingehalten werden. Die Verringerung des Gehalts an Kohlendioxid ist vielfach zur Einstellung eines vorgegebenen Brennwerts erforderlich.

Die Entfernung von Kohlendioxid aus Rauchgasen ist aus verschiedenen Gründen wünschenswert, insbesondere aber zur Verminderung der Emission von Kohlendioxid, die als Hauptursache für den so genannten Treibhauseffekt angesehen wird.

Zur Entfernung von Sauergasen werden Wäschen mit wässrigen Lösungen anorganischer oder organischer Basen eingesetzt. Beim Lösen von Sauergasen in dem Absorptionsmittel bilden sich mit den Basen Ionen. Das Absorptionsmittel kann durch Entspannen auf einen niedrigeren Druck und/oder Strippen regeneriert werden, wobei die ionischen Spezies zu Sauergasen zurück reagieren und/oder mittels Dampf abgestrippt werden. Nach dem Regenerationsprozess kann das Absorptionsmittel wiederverwendet werden.

Besonders bewährt hat sich auch das in dem US-Patent US 4,336,233 beschriebene Absorptionsmittel. Es handelt sich dabei um eine wässrige Lösung von Methyldiethanolamin (MDEA) und Piperazin als Absorptionsbeschleuniger oder Aktivator. Die beschriebene Waschflüssigkeit enthält 1,5 bis 4,5 mol/l Methyldiethanolamin (MDEA) und 0,05 bis 0,8 mol/l, bevorzugt bis zu 0,4 mol/l Piperazin.

Kohlenwasserstoffe sind zwar an sich wasserunlöslich. Aufgrund des hohen Gehalts an organischen Aminen weisen die wässrigen Aminlösungen, die zur Entfernung der sauren Gase eingesetzt werden, einen nicht zu vernachlässigenden lipophilen Charakter auf. Dieser führt zu einer Coabsorption von Kohlenwasserstoffen, wenn kohlenwasserstoffhaltige Fluide mit der wässrigen Aminlösung behandelt werden. Die Kohlenwasserstoff-Coabsorption ist aus verschiedenen Gründen nachteilig. Kohlenwasserstoffe stellen das primäre Wertprodukt vieler Fluidströme dar. Die Kohlenwasserstoff-Coabsorption führt daher zu einem unerwünschten Verlust an Wertprodukt. Zwar können die absorbierten Kohlenwasserstoffe in einer vorgelagerten Entspannungsstufe aus dem beladenen Absorptionsmittel freigesetzt werden, bevor das Absorptionsmittel regeneriert und von den sauren Gasen befreit wird. Dies bedingt aber einen zusätzlichen Verfahrensaufwand. In vielen Fällen ist auch die Reinheit des anfallenden Sauergases relevant. Z. B. kann der abgetrennte Schwefelwasserstoff in eine Sulfur Recovery Unit (SRU) geleitet werden, in der er katalytisch zu Schwefel oxidiert wird. Dabei ist die Anwesenheit von Kohlenwasserstoffen, insbesondere aromatischen Kohlenwasserstoffen wie Benzol, Toluol oder Xylol, schädlich, da sie die Qualität des erhaltenen Schwefels verschlechtern oder die in SRUs verwendeten Katalysatoren schädigen.

Rauchgase enthalten in der Regel erhebliche Mengen Sauerstoff. Bei der Kohlendioxid-Entfernung mit wässrigen Aminlösungen kann sich der Sauerstoff in gewissem Umfang in dem Absorptionsmittel physikalisch lösen. Der gelöste Sauerstoff kann, insbesondere unter den Bedingungen erhöhter Temperatur bei der Absorptionsmittel-Regeneration, zu einer allmähliche Zersetzung der Amine führen. Zur Vermeidung dieses Problems ist bereits vorgeschlagen worden, dem Absorptionsmittel Stabilisatoren gegen die Sauerstoff-induzierte Zersetzung zuzusetzen. Obgleich die Verwendung von Stabilisatoren die Zersetzung der Amine wirksam unterdrückt, ist ihr Einsatz mit erheblichen Kosten verbunden, da die Stabilisatormenge laufend ergänzt werden muss. Da die Aminzersetzung überwiegend durch den im Absorptionsmittel physikalisch gelösten Sauerstoff verursacht wird, wäre es wünschenswert, die Sauerstoff-Coabsorption im Absorptionsmittel zu unterdrücken.

Der Erfindung liegt die Aufgabe zugrunde, ein Verfahren und ein Absorptionsmittel zur Entfernung saurer Gase (Sauergase) aus kohlenwasserstoffhaltigen oder sauerstoffhaltigen Fluidströmen anzugeben, das eine verringerte Coabsorption von Kohlenwasserstoffen bzw. Sauerstoff zeigt, ohne (i) die Absorptionsgeschwindigkeit, mit der saure Gase absorbiert werden, wesentlich zu beeinträchtigen; (ii) ohne die Absorptionskapazität der Lösung für saure Gase wesentlich zu verringern und (iii) ohne den zur Regeneration erforderlichen Energiebedarf wesentlich zu erhöhen.

Es wurde nun gefunden, dass durch Mitverwendung von Metallsalzen einer Aminocarbonsäure und/oder Aminosulfonsäure in der wässrigen Lösung die Coabsorption von Kohlenwasserstoffen oder Sauerstoff ohne schädliche Auswirkungen auf Absorptionskapazität, Absorptionsgeschwindigkeit und Energiebedarf verringert werden kann.

Die Verwendung von Aminosäuresalzen in Absorptionsmitteln ist an sich bekannt. Die GB 1 543 748 beschreibt ein Verfahren zur Entfernung von CO₂ und H₂S aus einem Crackgas unter Verwendung einer wässrigen Lösung eines Alkalimetallsalzes einer N-Dialkyl-α-aminomonocarbonsäure, wie Dimethylglycin.

Die US-A 4,094,957 offenbart die Entfernung von CO₂ aus Gasströmen mit einer Absorptionslösung, die ein basisches Alkalimetallsalz, ein sterisch gehindertes Amin und eine Aminosäure wie N,N-Dimethylglycin, enthält.

EP-A 671 200 die Entfernung von CO₂ aus Verbrennungsgasen bei Atmosphärendruck mit einer wässrigen Lösung eines Aminosäuremetallsalzes und Piperazin.

Aus diesem Stand der Technik geht die Verwendung einer wässrigen Aminlösung, die ein Metallsalz einer Aminocarbonsäure und/oder Aminosulfonsäure umfasst, zur Entfernung saurer Gase aus kohlenwasserstoffhaltigen oder sauerstoffhaltigen Fluidströmen nicht hervor.

Die EP 0 125 358 A1 beschreibt ein Verfahren und eine Absorptionszusammensetzung zur Entfernung von CO₂ aus einem Gasstrom. Die Absorptionszusammensetzung enthält: (a) 10 bis etwa 40 Gew.-% eines Alkalimetallsalzes oder Hydroxids, (b) 2 bis etwa 20 Gew.-% einer nicht sterisch gehinderten Aminverbindung, (c) 2 bis etwa 20 Gew.-% einer sterisch gehinderten Aminosäure.

Das US-Patent Nr. 4,278,646 beschreibt ein Verfahren zur Entfernung von Schwefelwasserstoff aus Gasströmen durch Reduktion zu elementarem Schwefel. Dabei wird eine wässrige Lösung eingesetzt. Sie enthält durch eine Aminopolycarbonsäure chelatisierte Eisen(III) ionen bei einem pH-Wert von etwa 3,5 bis 5.

Die Erfindung betrifft ein Verfahren zum Entfernen saurer Gase aus einem kohlenwasserstoffhaltigen Fluidstrom oder einem sauerstoffhaltigen Fluidstrom, bei dem man den Fluidstrom mit einer wässrigen Lösung in Kontakt bringt, die weniger als 5 Gew.-% anorganische basische Salze enthält und (i) 2 bis 5 kmol / m³ wenigstens eines Alkanolamins und (ii) 0,5 bis 2 kmol / m³ wenigstens eines Metallsalzes einer Aminocarbonsäure, die eine N-Mono-C₁-C₄-alkyl-aminocarbonsäure oder eine N,N-Di-C₁-C₄-alkyl-aminocarbonsäure ist, umfasst; und aus dem mit den sauren Gasbestandteilen beladenen Absorptionsmittel die sauren Gasbestandteile in einem Regenerationsschritt freisetzt, wobei ein regeneriertes Absorptionsmittel erhalten wird.

Beschrieben ist auch ein Verfahren zum Entfernen saurer Gase aus einem kohlenwasserstoffhaltigen Fluidstrom oder einem sauerstoffhaltigen Fluidstrom, bei dem man den Fluidstrom mit einer wässrigen Lösung in Kontakt bringt, die von anorganischen basischen Salzen im Wesentlichen frei ist und (i) wenigstens ein Amin und (ii) wenigstens ein Metallsalz einer Aminocarbonsäure und/oder Aminosulfonsäure umfasst.

Die Erfindung betrifft außerdem ein Absorptionsmittel zum Entfernen saurer Gase aus einem Fluidstrom, die eine wässrige Lösung umfasst, die weniger als 5 Gew.-% anorganische basische Salze enthält und (i) 2 bis 5 kmol / m³ wenigstens eines Alkanolamins und (ii) 0,5 bis 2 kmol / m³ wenigstens eines Metallsalzes einer Aminocarbonsäure, die eine N-Mono-C₁-C₄-alkyl-aminocarbonsäure oder eine N,N-Di-C₁-C₄-alkyl-aminocarbonsäure ist, umfasst.

Beschreiben ist auch ein Absorptionsmittel zum Entfernen saurer Gase aus einem Fluidstrom, die eine wässrige Lösung umfasst, die von anorganischen basischen Salzen im Wesentlichen frei ist und (i) wenigstens ein Alkanolamin und (ii) ein Metallsalz einer Aminocarbonsäure und/oder Aminosulfonsäure umfasst.

Die folgenden Ausführungen in Bezug auf das erfindungsgemäße Verfahren gelten entsprechend für das erfindungsgemäße Absorptionsmittel und umgekehrt, soweit aus dem Kontext nicht anders ersichtlich.

Das Metallsalz der Aminocarbonsäure und/oder Aminosulfonsäure ist mit dem Amin, insbesondere dem Alkanolamin, in der wässrigen Lösung kompatibel und führt nicht zu Löslichkeits- oder Haltbarkeitsproblemen. Seine Mitverwendung verringert die Coabsorption von Kohlenwasserstoffen bzw. Sauerstoff, ohne die Absorptionsgeschwindigkeit, mit der saure Gase absorbiert werden, wesentlich zu beeinträchtigen; ohne die Absorptionskapazität der Lösung für saure Gase wesentlich zu verringern und ohne den zur Regeneration erforderlichen Energiebedarf wesentlich zu erhöhen.

Die hierin auch beschriebene wässrige Lösung ist von anorganischen basischen Salzen im Wesentlichen frei, d. h. sie enthält vorzugsweise weniger als etwa 10 Gew.-% anorganische basische Salze. Im erfindungsgemäßen Absorptionsmittel und Verfahren enthält sie weniger als 5 Gew.-% anorganische basische Salze. Anorganische basische Salze sind beispielsweise Alkalimetall- oder Erdalkalimetallcarbonate oder - hydrogencarbonate, wie insbesondere Kaliumcarbonat (Pottasche). Selbstverständlich kann man das Metallsalz der Aminocarbonsäure und/oder Aminosulfonsäure durch in situ-Neutralisation einer Aminocarbonsäure und/oder Aminosulfonsäure mit einer anorganischen Base wie Kaliumhydroxid erhalten; jedoch verwendet man hierzu keine wesentlich über die zur Neutralisation erforderliche Menge hinausgehenden Menge Base. Aminocarbonsäuren enthalten wenigstens eine Aminogruppe und wenigstens eine Carboxylgruppe in ihrer Molekülstruktur. Entsprechend enthalten Aminosulfonsäuren enthalten wenigstens eine Aminogruppe und wenigstens eine Sulfonsäuregruppe in ihrer Molekülstruktur.

### Geeignete Aminocarbonsäuren sind beispielsweise

α-Aminosäuren, wie Glycin (Aminoessigsäure), N-Methylglycin (N-Methylaminoessigsäure, Sarkosin), N,N-Dimethylglycin (Dimethylaminoessigsäure), N-Ethylglycin, N,N-Diethylclycin, Alanin (2-Aminopropionsäure), N-Methylalanin (2-(Methylamino)-propionsäure), N,N-Dimethylalanin, N-Ethylalanin, 2-Methylalanin (2-Aminoisobuttersäure), Leucin (2-Amino-4-methyl-pentan-1-säure), N-Methylleucin, N,N-Di-methylleucin, Isoleucin (1-Amino-2-methylpentansäure), N-Methylisoleucin, N,N-Di-methylisoleucin, Valin (2-Aminoisovaleriansäure), α-Methylvalin (2-Amino-2-methyl-isovaleriansäure), N-Methylvalin (2-Methylaminoisovaleriansäure), N,N-Dimethylvalin, Prolin (Pyrrolidin-2-carbonsäure), N-Methylprolin, Serin (2-Amino-3-hydroxy-propan-1-säure), N-Methylserin, N,N-Dimethylserin, 2-(Methylamino)-isobuttersäure, Piperidin-2-carbonsäure, N-Methyl-piperidin-2-carbonsäure,
β-Aminosäuren, wie 3-Aminopropionsäure (β-Alanin), 3-Methylaminopropionsäure, 3-Dimethylaminopropionsäure, Iminodipropionsäure, N-Methyliminodipropionsäure, Piperidin-3-carbonsäure, N-Methyl-piperidin-3-carbonsäure,
oder Aminocarbonsäuren wie Piperidin-4-carbonsäure, N-Methyl-piperidin-4-carbonsäure, 4-Aminobuttersäure, 4-Methylaminobuttersäure, 4-Dimethylaminobuttersäure.

### Geeignete Aminosulfonsäuren sind beispielsweise

Aminomethansulfonsäure, Taurin (2-Aminoethansulfonsäure), N-Methyltaurin.

Wenn die Aminocarbonsäure oder Aminosulfonsäure ein oder mehrere chirale Kohlenstoffatome aufweist, ist die Konfiguration unbeachtlich; es können sowohl die reinen Enantiomere/Diastereomere als auch beliebige Gemische oder Racemate verwendet werden.

Die Aminocarbonsäure ist vorzugsweise eine α-Aminosäure oder eine β-Aminosäure. Die Aminosulfonsäure ist vorzugsweise eine α-Aminosulfonsäure oder eine β-Aminosulfonsäure. Davon sind α-Aminosäure und β-Aminosulfonsäure besonders bevorzugt. Die Bezeichnung "α" bzw. "β" bedeutet in Übereinstimmung mit der üblichen Nomenklatur, dass die Aminogruppe durch ein bzw. zwei Kohlenstoffatome von der Carboxyl- oder Sulfonsäuregruppe getrennt ist.

Erfindungsgemäß eignen sich N-Mono-C₁-C₄-alkyl-aminocarbonsäuren und N,N-Di-C₁-C₄-alkyl-aminocarbonsäuren, insbesondere N-Mono-C₁-C₄-alkyl-α-aminocarbonsäuren und N,N-Di-C₁-C₄-alkyl-α-aminocarbonsäuren.

Das Metallsalz ist in der Regel ein Alkalimetall- oder Erdalkalimetallsalz, vorzugsweise ein Alkalimetallsalz, wie ein Natrium- oder Kaliumssalz, wovon Kaliumsalze am meisten bevorzugt sind.

Besonders bevorzugte Metallsalze von Aminocarbonsäuren sind das Kaliumsalz von Dimethylglycin oder N-Methylalanin.

Erfindungsgemäß enthält die wässrige Lösung 2 bis 5 kmol / m³, insbesondere 3,5 bis 4,5 kmol / m³ Alkanolamin (ausschließlich des Metallsalzes der Aminocarbonsäure) und 0,5 bis 2 kmol / m³, insbesondere 0,7 bis 1,5 kmol / m³ Metallsalz der Aminocarbonsäure.

Geeignete Amine sind primäre, sekundäre oder tertiäre Alkylamine und/oder Alkanolamine. Die erfindungsgemäß von der wässrigen Lösung umfassten Alkanolamine enthalten wenigstens ein Stickstoffatom, das durch wenigstens eine Hydroxyalkylgruppe, insbesondere eine C₂-C₃-Hydroxyalkylgruppe, meist eine 2-Hydroxyethyl-, 2-Hydroxpropyl- oder 3-Hydroxypropylgruppe, substituiert ist.

Erfindungsgemäß enthält die wässrige Lösung wenigstens ein Alkanolamin. Geeignete Alkanolamine sind ausgewählt unter Monoethanolamin (MEA), Diethanolamin (DEA), Diisopropanolamin, Triethanolamin (TEA), Diethylethanolamin (DEEA), Ethyldiethanolamin, Aminoethoxyethanol (AEE), Dimethylaminopropanol (DIMAP) und Methyldiethanolamin (MDEA), Methyldiisopropanolamin (MDIPA), 2-Amino-2-methyl-1-propanol (AMP), 2-Amino-1-butanol (2-AB) oder Gemischen davon.

In bevorzugten Ausführungsformen ist das Alkanolamin ein tertiäres Alkanolamin. Außerdem kann die wässrige Lösung einen oder mehrere Aktivatoren enthalten, die unter primären und sekundären Aminen ausgewählt sind. Die Verwendung von Aktivatoren erfolgt vorzugsweise, wenn die CO₂-Aufnahme durch intermediäre Bildung einer Carbamatstruktur beschleunigt werden soll.

Bei dem tertiären Alkanolamin kann es sich um ein Trialkanolamin, Alkyldialkanolamin oder Dialkylalkanolamin handeln. Die Alkylgruppen können geradkettig oder verzweigt sein und weisen im Allgemeinen ein bis vier Kohlenstoffatome auf. Die Alkanolgruppen weisen im Allgemeinen zwei bis vier Kohlenstoffatome auf. Beispiele für tertiäre Alkonolamine sind: Triethanolamin (TEA), Tributanolamin, Methyldiethanolamin (MDEA), Diethylethanolamin (DEEA), Dimethylethanolamin, Dimethylpropanolamin, Methyldiisopropanolamin (MDIPA), und vorzugsweise Methyldiethanolamin (MDEA).

Der Aktivator ist bevorzugt ausgewählt unter
a) 5- oder 6-gliedrigen gesättigten Heterocyclen mit wenigstens einer NH-Gruppe im Ring, die ein oder zwei weitere, unter Stickstoff und Sauerstoff ausgewählte Heteroatome im Ring enthalten können, oder
b) Verbindungen der Formel R¹-NH-R²-NH₂, worin R¹ für C₁-C₆-Alkyl steht und R² für C₂-C₆-Alkylen steht.

Beispiele bevorzugter Aktivatoren sind Piperazin, 2-Methylpiperazin, N-Methylpiperazin, Homopiperazin, Piperidin und Morpholin sowie 3-Methylaminopropylamin.

Weitere geeignete Aktivatoren sind Diethylentriamin, Triethylentetramin, Tetraethylenpentamin; 2,2-Dimethyl-1,3-diaminopropan, Hexamethylendiamin, 1,4-Diaminobutan, 3,3-Iminotrispropylamin, Tris(2-aminoethyl)amin, N-(2-Aminoethyl)piperazin, 2-(Ethylamino)ethanol, 2-(Methylamino)ethanol, 2-(n-Butylamino)ethanol, 2-Amino-1-butanol (2-AB), Aminoethoxyethanol, N-(2-Hydroxyethyl)ethylendiamin und N,N'-Bis(2-hydroxyethyl)ethylendiamin.

Besonders bevorzugte Kombinationen von tertiären Alkanolaminen und Aktivatoren sind die folgenden:
(i) Methyldiethanolamin und Piperazin,
(ii) Methyldiethanolamin und Methylaminopropylamin,
(iii) Methyldiethanolamin und Aminoethoxyethanol, oder
(iv) Methyldiethanolamin und 2-Amino-1-butanol.

Das Absorptionsmittel kann auch Additive, wie Korrosionsinhibitoren, Enzyme etc. enthalten. Im allgemeinen liegt die Menge an derartigen Additiven im Bereich von etwa 0,01-3 Gew.-% des Absorptionsmittels.

Das erfindungsgemäße Verfahren bzw. Absorptionsmittel ist geeignet zur Behandlung von Fluiden, insbesondere Gasströmen aller Art. Bei den sauren Gasen handelt es sich insbesondere um CO₂, H₂S, COS und Merkaptane. Außerdem können auch SO₃, SO₂, CS₂ und HCN entfernt werden. Fluide, welche die sauren Gase enthalten, sind einerseits Gase, wie Erdgas, Synthesegas, Koksofengas, Spaltgas, Kohlevergasungsgas, Kreisgas, Deponiegase und Verbrennungsgase, und andererseits mit dem Absorptionsmittel im Wesentlichen nicht mischbare Flüssigkeiten, wie LPG (Liquefied Petroleum Gas) oder NGL (Natural Gas Liquids). Das erfindungsgemäße Verfahren bzw. Absorptionsmittel ist besonders zur Behandlung von kohlenwasserstoffhaltigen Fluidströmen geeignet. Die enthaltenen Kohlenwasserstoffe sind z. B. aliphatische Kohlenwasserstoffe, wie C₁-C₄-Kohlenwasserstoffe, wie Methan, ungesättigte Kohlenwasserstoffe, wie Ethylen oder Propylen, oder aromatische Kohlenwasserstoffe wie Benzol, Toluol oder Xylol. Das erfindungsgemäße Verfahren bzw. Absorptionsmittel ist besonders zur Entfernung von CO₂ und H₂S geeignet.

Bei dem sauerstoffhaltigen Fluidstrom handelt es sich im Allgemeinen um einen Gasstrom, der auf folgende Weise gebildet wird:
a) Oxidation organischer Substanzen z. B. Verbrennungs- oder Rauchgase,
b) Kompostierung und Lagerung organische Substanzen enthaltender Abfallstoffe,
   oder
c) bakterielle Zersetzung organischer Substanzen.

Die Oxidation kann unter Flammenerscheinung, d. h. als herkömmliche Verbrennung, oder als Oxidation ohne Flammenerscheinung, z. B. in Form einer katalytischen Oxidation oder Partialoxidation, durchgeführt werden. Organische Substanzen, die der Verbrennung unterworfen werden, sind üblicherweise fossile Brennstoffe wie Kohle, Erdgas, Erdöl, Benzin, Diesel, Raffinate oder Kerosin, Biodiesel oder Abfallstoffe mit einem Gehalt an organischen Substanzen. Ausgangsstoffe der katalytischen (Partial-) Oxidation sind z. B. Methanol oder Methan, das zu Ameisensäure oder Formaldehyd umgesetzt werden kann.

Abfallstoffe, die der Oxidation, der Kompostierung oder Lagerung unterzogen werden, sind typischerweise Hausmüll, Kunststoffabfälle oder Verpackungsmüll.

Die Verbrennung der organische Substanzen erfolgt meistens in üblichen Verbrennungsanlagen mit Luft. Die Kompostierung und Lagerung organischer Substanzen enthaltender Abfallstoffe erfolgt im Allgemeinen auf Mülldeponien. Das Abgas bzw. die Abluft derartiger Anlagen kann vorteilhaft nach dem erfindungsgemäßen Verfahren behandelt werden.

Als organische Substanzen für bakterielle Zersetzung werden üblicherweise Stalldung, Stroh, Jauche, Klärschlamm, Fermentationsrückstände und dergleichen verwendet. Die bakterielle Zersetzung erfolgt z.B. in üblichen Biogasanlagen. Die Abluft derartiger Anlagen kann vorteilhaft nach dem erfindungsgemäßen Verfahren behandelt werden.

Das an sauren Gasbestandteilen reiche Ausgangsgas (Rohgas) wird in einem Absorptionsschritt in einem Absorber in Kontakt mit dem Absorptionsmittel gebracht, wodurch die sauren Gasbestandteile zumindest teilweise ausgewaschen werden.

Als Absorber fungiert vorzugsweise eine in üblichen Gaswäscheverfahren eingesetzte Waschvorrichtung. Geeignete Waschvorrichtungen sind beispielsweise Füllkörper, Packungs- und Bodenkolonnen, Membrankontaktoren, Radialstromwäscher, Strahlwäscher, Venturi-Wäscher und Rotations-Sprühwäscher, bevorzugt Packungs-, Füllkörper-und Bodenkolonnen, besonders bevorzugt Boden- und Füllkörperkolonnen. Die Behandlung des Fluidstroms mit dem Absorptionsmittel erfolgt dabei bevorzugt in einer Kolonne im Gegenstrom. Das Fluid wird dabei im Allgemeinen in den unteren Bereich und das Absorptionsmittel in den oberen Bereich der Kolonne eingespeist. In Bodenkolonnen sind Sieb-, Glocken- oder Ventilböden eingebaut, über welche die Flüssigkeit strömt. Füllkörperkolonnen können mit unterschiedlichen Formkörpern gefüllt werden. Wärme- und Stoffaustausch werden durch die Vergrößerung der Oberfläche aufgrund der meist etwa 25 bis 80 mm großen Formkörper verbessert. Bekannte Beispiele sind der Raschig-Ring (ein Hohlzylinder), Pall-Ring, Hiflow-Ring, Intalox-Sattel und dergleichen. Die Füllkörper können geordnet, aber auch regellos (als Schüttung) in die Kolonne eingebracht werden. Als Materialien kommen in Frage Glas, Keramik, Metall und Kunststoffe. Strukturierte Packungen sind eine Weiterentwicklung der geordneten Füllkörper. Sie weisen eine regelmäßig geformte Struktur auf. Dadurch ist es bei Packungen möglich, Druckverluste bei der Gasströmung zu reduzieren. Es gibt verschiedene Ausführungen von Packungen z. B. Gewebe- oder Blechpackungen. Als Material können Metall, Kunststoff, Glas und Keramik eingesetzt werden.

Die Temperatur des Absorptionsmittels beträgt im Absorptionsschritt im Allgemeinen etwa 30 bis 100 °C, bei Verwendung einer Kolonne beispielsweise 30 bis 70 °C am Kopf der Kolonne und 50 bis 100 °C am Boden der Kolonne. Der Gesamtdruck beträgt im Absorptionsschritt im Allgemeinen etwa 1 bis 120 bar, bevorzugt etwa 10 bis 100 bar.

Es wird ein an sauren Gasbestandteilen armes, d. h. ein an diesen Bestandteilen abgereichertes Produktgas (Reingas) und ein mit sauren Gasbestandteilen beladenes Absorptionsmittel erhalten. Das erfindungsgemäße Verfahren kann einen oder mehrere, insbesondere zwei, aufeinander folgende Absorptionsschritte umfassen. Die Absorption kann in mehreren aufeinander folgenden Teilschritten durchgeführt werden, wobei das die sauren Gasbestandteile enthaltende Rohgas in jedem der Teilschritte mit jeweils einem Teilstrom des Absorptionsmittels in Kontakt gebracht wird. Das Absorptionsmittel, mit dem das Rohgas in Kontakt gebracht wird, kann bereits teilweise mit sauren Gasen beladen sein, d. h. es kann sich beispielsweise um ein Absorptionsmittel, das aus einem nachfolgenden Absorptionsschritt in den ersten Absorptionsschritt zurückgeführt wurde, oder um teilregeneriertes Absorptionsmittel handeln. Bezüglich der Durchführung der zweistufigen Absorption wird Bezug genommen auf die Druckschriften EP-A 0 159 495, EP-A 0 20 190 434, EP-A 0 359 991 und WO 00100271.

Gemäß einer bevorzugten Ausführungsform wird das erfindungsgemäße Verfahren so durchgeführt, dass das die sauren Gase enthaltende Fluid zunächst in einem ersten Absorptionsschritt mit dem Absorptionsmittel bei einer Temperatur von 40 bis 100 °C, bevorzugt 50 bis 90 °C und insbesondere 60 bis 90 °C behandelt wird. Das an sauren Gasen abgereicherte Fluid wird dann in einem zweiten Absorptionsschritt mit dem Absorptionsmittel bei einer Temperatur von 30 bis 90 °C, bevorzugt 40 bis 80 °C und insbesondere 50 bis 80 °C, behandelt. Dabei ist die Temperatur um 5 bis 20 °C niedriger als in der ersten Absorptionsstufe.

Erfindungsgemäß werden aus dem mit den sauren Gasbestandteilen beladenen Absorptionsmittel die sauren Gasbestandteile in üblicher Weise (analog zu den nachfolgend zitierten Publikationen) in einem Regenerationsschritt freigesetzt, wobei ein regeneriertes Absorptionsmittel erhalten wird. Im Regenerationsschritt wird die Beladung des Absorptionsmittels verringert und das erhaltene regenerierte Absorptionsmittel wird vorzugsweise anschließend in den Absorptionsschritt zurückgeführt.

Im Allgemeinen beinhaltet der Regenerationsschritt mindestens eine Druckentspannung des beladenen Absorptionsmittels von einem hohen Druck, wie er bei der Durchführung des Absorptionsschritts herrscht, auf einen niedrigeren Druck. Die Druckentspannung kann beispielsweise mittels eines Drosselventils und/oder einer Entspannungsturbine geschehen. Die Regeneration mit einer Entspannungsstufe ist beispielsweise beschrieben in den Druckschriften US 4,537, 753 und US 4,553, 984.

Die Freisetzung der sauren Gasbestandteile im Regenerationsschritt kann beispielsweise in einer Entspannungskolonne, z.B. einem senkrecht oder waagerecht eingebauten Flash-Behälter oder einer Gegenstromkolonne mit Einbauten, erfolgen.

Bei der Regenerationskolonne kann es sich ebenfalls um eine Füllkörper-, Packungs- oder Bodenkolonne handeln. Die Regenerationskolonne weist am Sumpf einen Aufheizer auf, z. B. einen Zwangsumlaufverdampfer mit Umwälzpumpe. Am Kopf weist die Regenerationskolonne einen Auslass für die freigesetzten Sauergase auf. Mitgeführte Absorptionsmitteldämpfe werden in einem Kondensator kondensiert und in die Kolonne zurückgeführt.

Es können mehrere Entspannungskolonnen hintereinander geschaltet werden, in denen bei unterschiedlichen Drücken regeneriert wird. Beispielsweise kann in einer Vorentspannungskolonne bei hohem Druck, der typischerweise etwa 1,5 bar oberhalb des Partialdrucks der sauren Gasbestandteile im Absorptionsschritt liegt, und in einer Hauptentspannungskolonne bei niedrigem Druck, beispielsweise 1 bis 2 bar absolut, regeneriert werden. Die Regeneration mit zwei oder mehr Entspannungsstufen ist beschrieben in den Druckschriften US 4,537, 753, US 4,553, 984, EP-A 0 159 495, EP-A 0 202 600, EP-A 0 190 434 und EP-A 0 121 109.

Eine Verfahrensvariante mit zwei Niederdruckentspannungsstufen (1 bis 2 bar absolut), bei der die in der ersten Niederdruckentspannungsstufe teilregenerierte Absorptionsflüssigkeit erwärmt wird, und bei der gegebenenfalls vor der ersten Niederdruckentspannungsstufe eine Mitteldruckentspannungsstufe vorgesehen wird, bei der auf mindestens 3 bar entspannt wird, ist in DE 100 28 637 beschrieben. Dabei wird die beladene Absorptionsflüssigkeit zunächst in einer ersten Niederdruckentspannungsstufe auf einen Druck von 1 bis 2 bar (absolut) entspannt. Anschließend wird die teilregenerierte Absorptionsflüssigkeit in einem Wärmetauscher erwärmt und dann in einer zweiten Niederdruckentspannungsstufe erneut auf einen Druck von 1 bis 2 bar (absolut) entspannt.

Die letzte Entspannungsstufe kann auch unter Vakuum durchgeführt werden, das beispielsweise mittels eines Wasserdampfstrahlers, gegebenenfalls in Kombination mit einem mechanischen Vakuumerzeugungsapparat, erzeugt wird, wie beschrieben in EP-A 0 159 495, EP-A 0 202 600, EP-A 0 190 434 und EP-A 0 121 109(US 4, 551, 158).

Wegen der optimalen Abstimmung des Gehalts an den Aminkomponenten weist das erfindungsgemäße Absorptionsmittel eine hohe Beladbarkeit mit sauren Gasen auf, die auch leicht wieder desorbiert werden können. Dadurch können bei dem erfindungsgemäßen Verfahren der Energieverbrauch und der Lösungsmittelumlauf signifikant reduziert werden.

Die Erfindung wird anhand der beigefügten Zeichnung und des nachfolgenden Beispiels näher veranschaulicht.

Figur 1 zeigt die Löslichkeit von Methan in Abhängigkeit vom Druck in einem erfindungsgemäßen Absorptionsmittel und in einem Vergleichslösungsmittel ohne Aminosäuresalz.

Die Figuren 2 und 3 zeigen schematisch eine zur Durchführung des erfindungsgemäßen Verfahrens geeignete Vorrichtung.

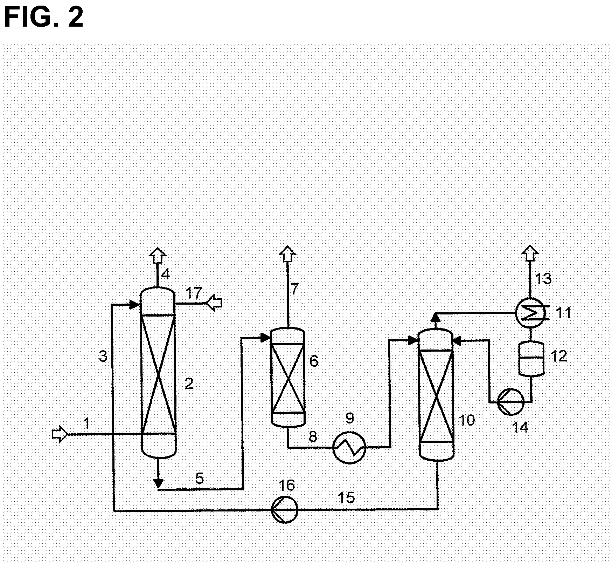

In Figur 2 ist schematisch eine Vorrichtung dargestellt, bei der die Absorptionsstufe einstufig und die Entspannungsstufe zweistufig durchgeführt werden. Das Feedgas wird über Leitung 1 in den unteren Bereich des Absorbers 2 eingespeist. Beim Absorber 2 handelt es sich um eine Kolonne, die mit Füllkörpern gepackt ist, um den Massen- und Wärmeaustausch zu bewirken. Das Absorptionsmittel, bei dem es sich um regeneriertes Absorptionsmittel mit einem geringen Restgehalt an sauren Gasen handelt, wird über die Leitung 3 auf den Kopf des Absorbers 2 im Gegenstrom zu dem Feedgas aufgegeben. Das an sauren Gasen abgereicherte Gas verlässt den Absorber 2 über Kopf (Leitung 4). Das mit sauren Gasen angereicherte Absorptionsmittel verlässt den Absorber 2 am Boden über Leitung 5 und wird in den oberen Bereich der Hochdruck-Entspannungskolonne 6 eingeleitet, die im Allgemeinen bei einem Druck betrieben wird, der oberhalb des Sauergas-Partialdrucks des dem Absorber zugeführten Rohgases liegt. Die Entspannung des Absorptionsmittels erfolgt im Allgemeinen mit Hilfe üblicher Vorrichtungen, beispielsweise eines Stand-Regelventils, einer hydraulischen Turbine oder einer umgekehrt laufenden Pumpe. Bei der Entspannung wird der größte Teil der gelösten nicht-sauren Gase sowie ein kleiner Teil der sauren Gase freigesetzt. Diese Gase werden über Leitung 7 aus der Hochdruck-Entspannungskolonne 6 über Kopf ausgeschleust.

Das Absorptionsmittel, das nach wie vor mit dem Großteil der sauren Gase beladen ist, verlässt die Hochdruck-Entspannungskolonne über Leitung 8 und wird im Wärmetauscher 9 aufgeheizt, wobei ein kleiner Teil der sauren Gase freigesetzt werden kann.

Das aufgeheizte Absorptionsmittel wird in den oberen Bereich einer Niederdruck- Entspannungskolonne 10 eingeleitet, die mit einer Füllkörperpackung ausgerüstet ist, um eine große Oberfläche zu erzielen und so die Freisetzung der Sauergase und die Einstellung des Gleichgewichts zu bewirken. In der Niederdruck-Entspannungskolonne 10 werden der größte Teil der Sauergase praktisch vollständig durch Flashen freigesetzt. Das Absorptionsmittel wird auf diese Weise gleichzeitig regeneriert und abgekühlt. Am Kopf der Niederdruck-Entspannungskolonne 10 ist ein Rückflusskühler 11 mit einem Auffangbehälter 12 vorgesehen, um die freigesetzten sauren Gase zu kühlen und einen Teil des Dampfes zu kondensieren. Die Hauptmenge des sauren Gases verlässt den Rückflusskühler 11 über Leitung 13. Das Kondensat wird mittels Pumpe 14 auf den Kopf der Niederdruck-Entspannungskolonne 10 zurückgepumpt. Das regenerierte Absorptionsmittel, das noch einen geringen Teil der Sauergase enthält, verlässt die Niederdruck-Entspannungskolonne 10 am Boden über Leitung 15 und wird mittels Pumpe 16 über Leitung 3 auf den Kopf des Absorbers 2 aufgegeben. Über Leitung 17 kann Frischwasser zum Ausgleich des mit den Gasen ausgetragenen Wassers eingespeist werden.

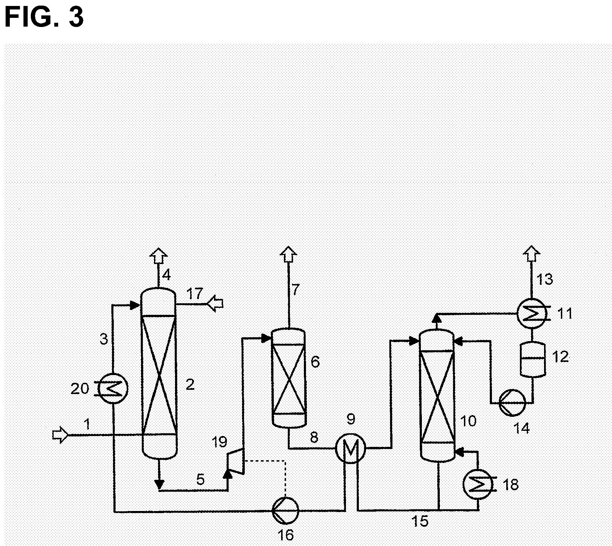

Figur 3 zeigt schematisch eine Vorrichtung zur Durchführung des erfindungsgemäßen Verfahrens mit einer Entspannungsstufe und einer Desorptionsstufe (Stripper).

Das Feedgas wird über Leitung 1 in den unteren Bereich des Absorbers 2 eingespeist. Das Absorptionsmittel wird über die Leitung 3 auf den Kopf des Absorbers 2 im Gegenstrom zu dem Feedgas aufgegeben. Das an sauren Gasen abgereicherte Gas verlässt den Absorber 2 über Kopf (Leitung 4). Das mit sauren Gasen angereicherte Absorptionsmittel verlässt den Absorber 2 am Boden über Leitung 5 und wird über eine Entspannungsturbine 19 in den oberen Bereich der Hochdruck-Entspannungskolonne 6 eingeleitet, die im Allgemeinen bei einem Druck betrieben wird, der oberhalb des CO₂-Partialdrucks des dem Absorber zugeführten Rohgases liegt. Bei der Entspannung wird der größte Teil der gelösten nicht-sauren Gase sowie ein kleiner Teil der sauren Gase frei- gesetzt. Diese Gase werden über Leitung 7 aus der Hochdruck-Entspannungskolonne 6 über Kopf ausgeschleust. Die an der Entspannungsturbine 19 anfallende Energie kann zum Betrieb der Pumpe 16 verwendet werden.

Das Absorptionsmittel, das nach wie vor mit dem Großteil der sauren Gase beladen ist, verlässt die Hochdruck-Entspannungskolonne über Leitung 8 und wird im Wärmetauscher 9 durch indirekten Wärmetausch mit regeneriertem Absorptionsmittel aufgeheizt, das über die Leitung 15 herangeführt wird.

Das aufgeheizte beladene Absorptionsmittel wird in den oberen Bereich einer Desorberkolonne 10 eingeleitet. Die Kolonne 10 verfügt über eine indirekte Sumpfbeheizung über den Wärmetauscher 18. In der Kolonne 10 werden ein Teil des CO₂ und H₂S durch Flashen freigesetzt, der Rest wird im unteren Teil der Kolonne 10 praktisch vollständig durch Strippen ausgetrieben. Am Kopf der Kolonne 10 ist ein Rückflusskühler 11 mit einem Auffangbehälter 12 vorgesehen, um die freigesetzten sauren Gase zu kühlen und einen Teil des Dampfes zu kondensieren. Die Hauptmenge des sauren Gases verlässt den Rückflusskühler 11 über Leitung 13. Das Kondensat wird mittels Pumpe 14 auf den Kopf der Kolonne 10 zurückgepumpt. Das regenerierte Absorptionsmittel verlässt die Kolonne 10 am Boden über Leitung 15 und wird über den Wärmetauscher 9 mittels Pumpe 16 über Leitung 3 und den Kühler 20 auf den Kopf des Absorbers 2 aufgegeben. Über Leitung 17 kann Frischwasser zum Ausgleich des mit den Gasen ausgetragenen Wassers eingespeist werden.

### Beispiel

Man verwendete ein Absorptionsmittel folgender Zusammensetzung: 40 Gew.-% Methyldiethanolamin und 60 Gew.-% Wasser. Dieses Absorptionsmittel diente als Vergleichsabsorptionsmittel.

Ein erfindungsgemäßes Absorptionsmittel wurde erhalten, indem man das Vergleichsabsorptionsmittel mit 10 Gew.-% Kaliumsalz von Dimethylglycin versetzte.

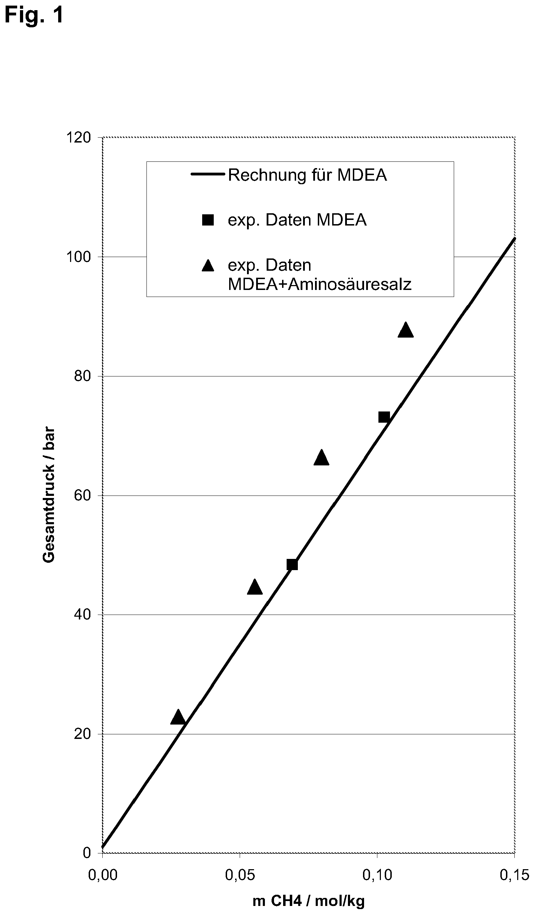

Beide Absorptionsmittel wurden mit 20 Nm³ Kohlendioxid /t Absorptionsmittel vorbeladen. Dann bestimmte man die Löslichkeit von Methan in beiden Absorptionsmitteln bei verschiedenen Methan-Partialdrücken, indem man. Die Ergebnisse sind in der Fig. 1 dargestellt.

Man sieht, dass im erfindungsgemäßen Absorptionsmittel bei gleichem Methan-Partialdruck deutlich weniger Methan löslich ist. Es wird erwartet, dass die Reduzierung der Löslichkeit bei längerkettigen und ungesättigten Kohlenwasserstoffen noch ausgeprägter ist als bei Methan.

## Patentansprüche

1. Verfahren zum Entfernen saurer Gase aus einem kohlenwasserstoffhaltigen Fluidstrom oder einem sauerstoffhaltigen Fluidstrom, bei dem man den Fluidstrom mit einer wässrigen Lösung in Kontakt bringt, die weniger als 5 Gew.-% anorganische basische Salze enthält und (i) 2 bis 5 kmol / m³ wenigstens eines Alkanolamins und (ii) 0,5 bis 2 kmol / m³ wenigstens eines Metallsalzes einer Aminocarbonsäure, die eine N-Mono-C₁-C₄-alkyl-aminocarbonsäure oder eine N,N-Di-C₁-C₄-alkyl-aminocarbonsäure ist, umfasst; und aus dem mit den sauren Gasbestandteilen beladenen Absorptionsmittel die sauren Gasbestandteile in einem Regenerationsschritt freisetzt, wobei ein regeneriertes Absorptionsmittel erhalten wird.

2. Verfahren nach Anspruch 1, bei dem das Metallsalz der Aminocarbonsäure das Kaliumsalz von N,N-Dimethylglycin oder N-Methylalanin ist.

3. Verfahren nach Anspruch 1 oder 2, bei dem das Alkanolamin ausgewählt ist unter Monoethanolamin (MEA), Diethanolamin (DEA), Diisopropanolamin, Triethanolamin (TEA), Diethylethanolamin (DEEA), Ethyldiethanolamin, Aminoethoxyethanol (AEE), Dimethylaminopropanol (DIMAP), Methyldiethanolamin (MDEA), Methyldiisopropanolamin (MDIPA), 2-Amino-2-methyl-1-propanol (AMP), 2-Amino-1-butanol (2-AB) oder Gemischen davon.

4. Verfahren nach Anspruch 1, bei dem das Alkanolamin ein tertiäres Alkanolamin ist.

5. Verfahren nach Anspruch 4, bei dem die wässrige Lösung außerdem einen oder mehrere Aktivatoren enthält.

6. Verfahren nach Anspruch 5, bei dem der Aktivator ausgewählt ist unter Piperazin, Methylaminopropylamin, Aminoethoxyethanol und 2-Amino-1-butanol.

7. Verfahren nach Anspruch 5, bei dem die wässrige Lösung umfasst:
(i) Methyldiethanolamin und Piperazin,
(ii) Methyldiethanolamin und Methylaminopropylamin,
(iii) Methyldiethanolamin und Aminoethoxyethanol, oder
(iv) Methyldiethanolamin und 2-Amino-1-butanol.

8. Absorptionsmittel zum Entfernen saurer Gase aus einem Fluidstrom, umfassend eine wässrige Lösung, die weniger als 5 Gew.-% anorganische basische Salze enthält und (i) 2 bis 5 kmol / m³ wenigstens eines Alkanolamins und (ii) 0,5 bis 2 kmol / m³ wenigstens eines Metallsalzes einer Aminocarbonsäure, die eine N-Mono-C₁-C₄-alkyl-aminocarbonsäure oder eine N,N-Di-C₁-C₄-alkyl-aminocarbonsäure ist, umfasst.

9. Absorptionsmittel nach Anspruch 8, bei dem das Metallsalz der Aminocarbonsäure das Kaliumsalz von N,N-Dimethylglycin oder N-Methylalanin ist.

10. Absorptionsmittel nach Anspruch 8 oder 9, bei dem das Alkanolamin ausgewählt ist unter Monoethanolamin (MEA), Diethanolamin (DEA), Diisopropanolamin, Triethanolamin (TEA), Diethylethanolamin (DEEA), Ethyldiethanolamin, Aminoethoxyethanol (AEE), Dimethylaminopropanol (DIMAP), Methyldiethanolamin (MDEA), Methyldiisopropanolamin (MDIPA), 2-Amino-2-methyl-1-propanol (AMP), 2-Amino-1-butanol (2-AB) oder Gemischen davon.

11. Absorptionsmittel nach einem der Ansprüche 8 bis 10, bei dem das Alkanolamin ein tertiäres Alkanolamin ist.

12. Absorptionsmittel nach Anspruch 11, bei dem die wässrige Lösung außerdem einen oder mehrere Aktivatoren enthält.

13. Absorptionsmittel nach Anspruch 12, bei dem die Aktivatoren ausgewählt sind unter Piperazin, Methylaminopropylamin, Aminoethoxyethanol und 2-Amino-1-butanol.

## Claims

1. A process for removing acid gases from a hydrocarbonaceous fluid stream or an oxygen-comprising fluid stream in which the fluid stream is contacted with an aqueous solution which comprises less that 5% by weight of inorganic basic salts and comprises (i) 2 to 5 kmol/m³ of at least one alkanolamine and (ii) 0.5 to 2 kmol/m³ of at least one metal salt of an aminocarboxylic acid is a N-mono-C₁-C₄-alkylaminocarboxylic acid or a N,N-di-C₁-C₄-alkylaminocarboxylic acid; and the acid gas components are released from the absorption medium loaded with the acid gas components in a regeneration step, a generated absorption medium is being obtained.

2. The process according to claim 1, wherein the metal salt of the aminocarboxylic acid is the potassium salts of N,N-dimethylglycine or N-methylalanine.

3. The process according to claim 1 or 2, wherein the alkanolamine is selected from monoethanolamine (MEA), diethanolamine (DEA), diisopropanolamine, triethanolamine (TEA), diethylethanolamine (DEEA), ethyldiethanolamine, aminoethoxyethanol (AEE), dimethylaminopropanol (DIMAP), methyldiethanolamine (MDEA), methyldiisopropanolamine (MDIPA), 2-amino-2-methyl-1-propanol (AMP), 2-amino-1-butanol (2-AB) or mixtures thereof.

4. The process according to claim 1, wherein the alkanolamine is a tertiary alkanolamine.

5. The process according to claim 4, in which the aqueous solution in addition comprises one or more activators.

6. The process according to claim 5, wherein the activator is selected from piperazine, methylaminopropylamine, aminoethoxyethanol and 2-amino-1-butanol.

7. The process according to claim 5, wherein the aqueous solution comprises:
(i) methyldiethanolamine and piperazine,
(ii) methyldiethanolamine and methylaminopropylamine,
(iii) methyldiethanolamine and aminoethoxyethanol, or
(iv) methyldiethanolamine and 2-amino-1-butanol.

8. An absorption medium for removing acid gases from a fluid stream, comprising an aqueous solution which comprises less than 5% by weight of inorganic basic salts and comprises (i) 2 to 5 kmol/m³ of at least one alkanolamine and (ii) 0.5 to 2 kmol/m³ of at least one metal salt of an aminocarboxylic acid which is a N-mono-C₁-C₄-alkylaminocarboxylic acid or a N,N-di-C₁-C₄-alkylaminocarboxylic acid.

9. The absorption medium according to claim 8, wherein the metal salt of the aminocarboxylic acid is the potassium salt of N,N-dimethylglycine or N-methylalanine.

10. The absorption medium according to claim 8 or 9, wherein the alkanolamine is selected from monoethanolamine (MEA), diethanolamine (DEA), diisopropanolamine, triethanolamine (TEA), diethylethanolamine (DEEA), ethyldiethanolamine, aminoethoxyethanol (AEE), dimethylaminopropanol (DIMAP), methyldiethanolamine (MDEA), methyldiisopropanolamine (MDIPA), 2-amino-2-methyl-1-propanol (AMP), 2-amino-1-butanol (2-AB) or mixtures thereof.

11. The absorption medium according to one of claims 8 to 10, wherein the alkanolamine is a tertiary alkanolamine.

12. The absorption medium according to claim 11, wherein the aqueous solution in addition comprises one or more activators.

13. The absorption medium according to claim 12, wherein the activators are selected from piperazine, methylaminopropylamine, aminoethoxyethanol and 2-amino-1-butanol.

## Revendications

1. Procédé d'élimination de gaz acides d'un courant fluide contenant des hydrocarbures ou d'un courant fluide contenant de l'oxygène, dans lequel le courant fluide est mis en contact avec une solution aqueuse, qui contient moins de 5 % en poids de sels basiques inorganiques et comprend (i) 2 à 5 kmol/m³ d'au moins une alcanolamine et (ii) 0,5 à 2 kmol/m³ d'au moins un sel métallique d'un acide aminocarboxylique, qui est un acide N-mono-alkyle en C₁-C₄-aminocarboxylique ou un acide N,N-di-alkyle en C₁-C₄-aminocarboxylique ; et les constituants gazeux acides sont libérés de l'agent d'absorption chargé avec les constituants gazeux acides dans une étape de régénération, un agent d'absorption régénéré étant obtenu.

2. Procédé selon la revendication 1, dans lequel le sel métallique de l'acide aminocarboxylique est le sel de potassium de N,N-diméthylglycine ou de N-méthylalanine.

3. Procédé selon la revendication 1 ou 2, dans lequel l'alcanolamine est choisie parmi la monoéthanolamine (MEA), la diéthanolamine (DEA), la diisopropanolamine, la triéthanolamine (TEA), la diéthyléthanolamine (DEEA), l'éthyldiéthanolamine, l'amino-éthoxyéthanol (AEE), le diméthylaminopropanol (DIMAP), la méthyldiéthanolamine (MDEA), la méthyldüsopropanolamine (MDIPA), le 2-amino-2-méthyl-1-propanol (AMP), le 2-amino-1-butanol (2-AB) ou leurs mélanges.

4. Procédé selon la revendication 1, dans lequel l'alcanolamine est une alcanolamine tertiaire.

5. Procédé selon la revendication 4, dans lequel la solution aqueuse contient en outre un ou plusieurs activateurs.

6. Procédé selon la revendication 5, dans lequel l'activateur est choisi parmi la pipérazine, la méthylaminopropylamine, l'aminoéthoxyéthanol et le 2-amino-1-butanol.

7. Procédé selon la revendication 5, dans lequel la solution aqueuse comprend :
(i) de la méthyldiéthanolamine et de la pipérazine,
(ii) de la méthyldiéthanolamine et de la méthylaminopropylamine,
(iii) de la méthyldiéthanolamine et de l'aminoéthoxyéthanol, ou
(iv) de la méthyldiéthanolamine et du 2-amino-1-butanol.

8. Agent d'absorption pour l'élimination de gaz acides d'un courant fluide, comprenant une solution aqueuse, qui contient moins de 5 % en poids de sels basiques inorganiques et comprend (i) 2 à 5 kmol/m³ d'au moins une alcanolamine et (ii) 0,5 à 2 kmol/m³ d'au moins un sel métallique d'un acide aminocarboxylique, qui est un acide N-mono-alkyle en C₁-C₄-aminocarboxylique ou un acide N,N-di-alkyle en C₁-C₄-aminocarboxylique.

9. Agent d'absorption selon la revendication 8, dans lequel le sel métallique de l'acide aminocarboxylique est le sel de potassium de N,N-diméthylglycine ou de N-méthylalanine.

10. Agent d'absorption selon la revendication 8 ou 9, dans lequel l'alcanolamine est choisie parmi la monoéthanolamine (MEA), la diéthanolamine (DEA), la diisopropanolamine, la triéthanolamine (TEA), la diéthyléthanolamine (DEEA), l'éthyldiéthanolamine, l'amino-éthoxyéthanol (AEE), le diméthylaminopropanol (DIMAP), la méthyldiéthanolamine (MDEA), la méthyldiisopropanolamine (MDIPA), le 2-amino-2-méthyl-1-propanol (AMP), le 2-amino-1-butanol (2-AB) ou leurs mélanges.

11. Agent d'absorption selon l'une quelconque des revendications 8 à 10, dans lequel l'alcanolamine est une alcanolamine tertiaire.

12. Agent d'absorption selon la revendication 11, dans lequel la solution aqueuse contient en outre un ou plusieurs activateurs.

13. Agent d'absorption selon la revendication 12, dans lequel les activateurs sont choisis parmi la pipérazine, la méthylaminopropylamine, l'aminoéthoxyéthanol et le 2-amino-1-butanol.
